# EUROPÄISCHE PATENTSCHRIFT

(11) **EP 2 922 580 B1**
(45) Veröffentlichungstag und Bekanntmachung des Hinweises auf die Patenterteilung: **16.11.2016**
(21) Anmeldenummer: 13792008.8
(22) Anmeldetag: 18.11.2013
(51) Int. Cl.: A61L 15/60, C07C 51/215, C07C 51/25, C08F 220/06

(54) **VERFAHREN ZUR HERSTELLUNG VON SUPERABSORBERN AUF BASIS NACHWACHSENDER ROHSTOFFE**
METHOD FOR PRODUCING SUPERABSORBERS BASED ON RENEWABLE RAW MATERIALS
PROCÉDÉ DE PRÉPARATION DE SUPER-ABSORBANTS À BASE DE MATIÈRES PREMIÈRES RENOUVELABLES

(30) Priorität: 26.11.2012 US 201261729645 P; 26.11.2012 EP 12194209
(43) Veröffentlichungstag der Anmeldung: 30.09.2015
(73) Patentinhaber: BASF SE, 67056 Ludwigshafen am Rhein (DE)
(72) Erfinder: HÖRNER, Klaus Dieter, 68623 Lampertheim (DE); SCHRÖDER, Jürgen, 67071 Ludwigshafen (DE); FUNK, Rüdiger, 65527 Niedernhausen (DE); WÜSTEFELD, Renate, 67346 Speyer (DE)
(74) Vertreter: BASF IP Association
(86) Internationale Anmeldenummer: PCT/EP2013/074007
(87) Internationale Veröffentlichungsnummer: WO 2014/079785

(56) Entgegenhaltungen:
- WO-A2-2006/092272
- US-A1- 2006 004 229
- STEVEN P PYL ET AL: "Biomass to olefins: Cracking of renewable naphtha", CHEMICAL ENGINEERING JOURNAL, ELSEVIER SEQUOIA, LAUSANNE, CH, Bd. 176, 12. April 2011 (2011-04-12), Seiten 178-187, XP028115203, ISSN: 1385-8947, DOI: 10.1016/J.CEJ.2011.04.062 [gefunden am 2011-05-19]

## Beschreibung

Die vorliegende Erfindung betrifft ein Verfahren zur Herstellung von wasserabsorbierenden Polymerpartikeln, umfassend die Schritte thermische Spaltung von Bio-Naphtha in Gegenwart von Wasserdampf, Abtrennung von Propen und zumindest eines Teiles des Propans, Gashasenoxidation zu Acrylsäure und Polymerisation zu wasserabsorbierenden Polymerpartikeln. Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm.² (AUL0.3psi) durchläuft ein Maximum.

EP 2 395 029 A1 offenbart die Verwendung einer Acrylsäure, enthaltend mindestens 0,04 Gew.-% Propionsäure, zur Herstellung wasserabsorbierender Polymerpartikel. Die Beispiele zeigen, dass die Eigenschaften der erhaltenen wasserabsorbierenden Polymerartikel, beispielsweise die Zentrifugenretentionskapazität (CRC), durch die Gegenwart von Propionsäure bei der Polymerisation verbessert werden können.

DE 103 36 386 A1 offenbart ein Verfahren zur Herstellung von Acrylsäure durch zweistufige Gasphasenoxidation von Propen und die anschließende Aufarbeitung des Reaktionsgemisches.

EP 2 290 045 A1 und Steven P. Pyl et al., Chem. Eng. J. 176-177(2011), 178-187 offenbaren die Herstellung von Propen bzw. Olefinen aus Bio-Naphtha.

WO 2006/092272 A2 beschreibt ein Verfahren zur Herstellung von auf nachwachsenden Rohstoffen basierender Acrylsäure und wasserabsorbierenden Polymeren.

Aufgabe der vorliegenden Erfindung war die Bereitstellung eines verbesserten Verfahrens zur Herstellung wasserabsorbierender Polymerpartikel.

Eine weitere Aufgabe der vorliegenden Erfindung war die Bereitstellung eines kostengünstigen Verfahrens zur Herstellung von wasserabsorbierenden Polymerpartikeln auf Basis nachwachsender Rohstoffe.

Gelöst wurde die Aufgabe durch ein Verfahren zur Herstellung von wasserabsorbierenden Polymerpartikeln, umfassend die Schritte
i) thermische Spaltung von Bio-Naphtha, basierend auf natürlichen Ölen und/oder Fetten, in Gegenwart von Wasserdampf zu einem Gemisch , enthaltend Propan und Propen,
ii) Abtrennung von Propen und zumindest eines Teiles des Propans aus dem in Schritt i) erhaltenen Gemisch,
iii) Gashasenoxidation der in Schritt ii) erhaltenen Propen/Propan-Mischung zu Acrylsäure und
iv) Polymerisation der in Schritt iii) erhalten Acrylsäure zu wasserabsorbierenden Polymerpartikeln.

Bio-Naphtha im Sinne dieser Erfindung sind alle natürlichen Öle und/oder Fette sowie deren Derivate. So ist es möglich die natürlichen Öle und/oder Fette zu verseifen und nur die so erhaltenen Fettsäuren als Bio-Naphtha ein zu setzten, wie in EP 2 290 034 A1 beschrieben. Es ist aber auch möglich die abgetrennten Fettsäuren zu hydrieren, wie in EP 2 290 045 A1 beschrieben. In einer besonders bevorzugten Ausführungsform der vorliegenden Erfindung wird Bio-Naphtha auf Basis von Palmöl eingesetzt.

Die Gasphasenoxidation von Propen zu Acrylsäure unterliegt keinen Beschränkungen und wird vorzugsweise zweistufig durchgeführt, d.h. in einer ersten Stufe von Propen zu Acrolein und in einer zweiten Stufe von Acrolein zu Acrylsäure.

Der vorliegenden Erfindung liegt die Erkenntnis zu Grunde, dass bei der thermischen Spaltung von Bio-Naphtha, bezogen auf Propen, mehr Propan entsteht. Aufgrund des ähnlichen Siedepunktes ist die Abtrennung von Propan aus Propen aufwendig und teuer.

Wird nun Propen in einer Gasphasenoxidation zu Acrylsäure umgesetzt, so wird unter diesen Bedingungen im Propen enthaltendes Propan zu Propionsäure oxidiert. Die Abtrennung von Propionsäure aus Acrylsäure ist aufgrund ähnlicher Siedepunkte ebenfalls aufwendig und teuer.

Zur Verbesserung der Produkteigenschaften ist die Gegenwart von Propionsäure bei der Herstellung von wasserabsorbierenden Polymerpartikeln durchaus erwünscht. Die verwendete Acrylsäure enthält vorzugsweise von 0,02 bis 2,0 Gew.-%, besonders bevorzugt von 0,03 bis 1,0 Gew.-%, ganz besonders bevorzugt von 0,04 bis 0,5 Gew.-%, Propionsäure.

Daher kann bei Verwendung von Acrylsäure auf Basis von Bio-Naphtha auf eine besonders aufwendige Reinigung des Propens oder der Acrylsäure verzichtet werden.

Das zur Gasphasenoxidation eingesetzte Propen enthält vorzugsweise von 3,4 bis 30 Gew.-%, besonders bevorzugt von 3,8 bis 15 Gew.-%, ganz besonders bevorzugt von 4,2 bis 7,5 Gew.-%, Propan, jeweils bezogen auf Propen.

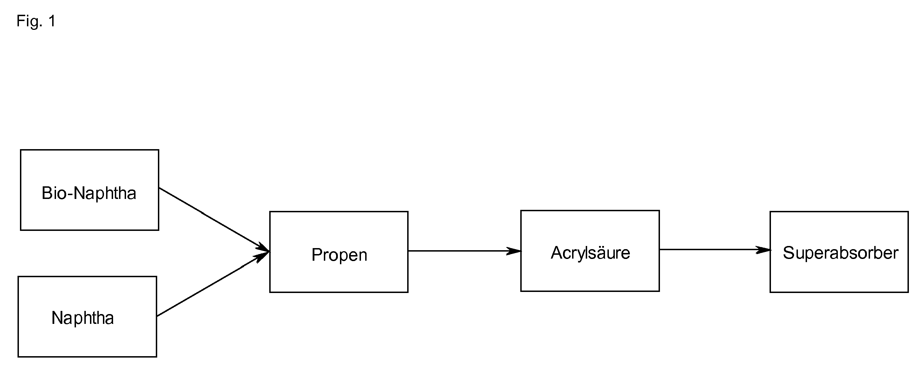

Es ist auch möglich durch gleichzeitige Verwendung von Bio-Naphtha und Naphtha den Gehalt an Propionsäure auf den gewünschten Wert einzustellen, beispielsweise durch gemeinsame Verwendung von Bio-Naphtha und Naphtha bei der thermischen Spaltung in Schritt 1 (Fig. 1).

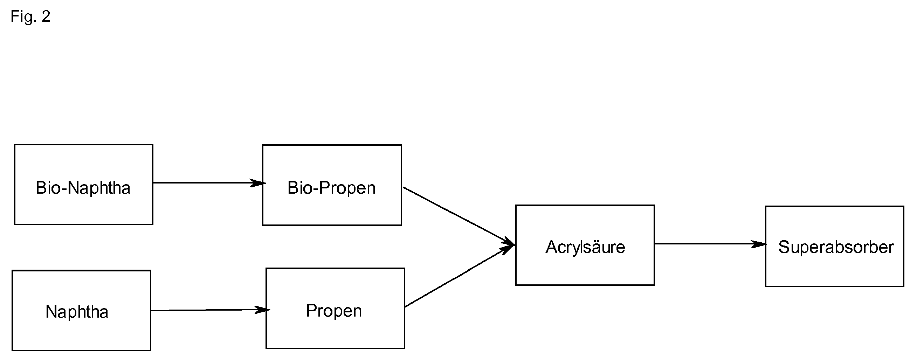

Es ist aber auch möglich Bio-Naphtha und Naphtha getrennt zu Propen umzusetzen und das so erhaltene Bio-Propen und Propen gemeinsam zu Acrylsäure umzusetzen (Fig. 2).

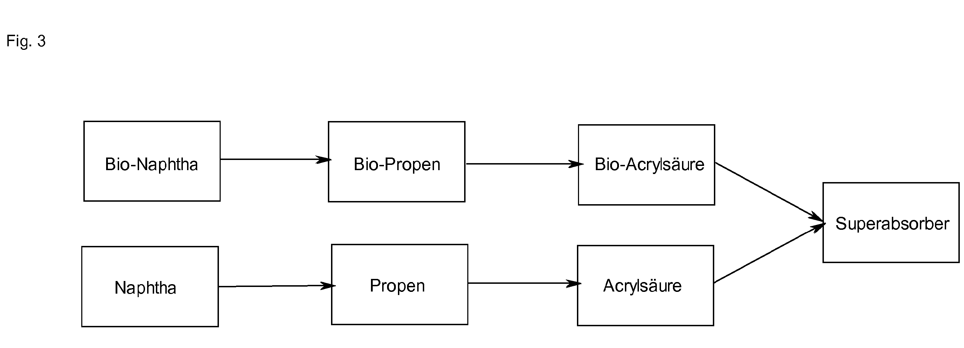

Weiterhin ist es möglich Bio-Naphtha und Naphtha getrennt zu Propen, das so erhaltene Bio-Propen und Propen getrennt zu Acrylsäure und die so erhaltene Bio-Acrylsäure und Acrylsäure gemeinsam zu wasserabsorbierenden Polymerpartikeln umzusetzen (Fig. 3).

Weiterhin ist es möglich nur einen Teil der Einsatzmenge bei der thermischen Spaltung von Naphtha (Steamcracking) auf Bio-Naphtha umzustellen, beispielsweise auf genau die Menge, die rechnerisch über die Stufen des Propens und der Acrylsäure zur Herstellung der wasserabsorbierenden Polymerpartikel notwendig ist. So können auch kleine Mengen an wasserabsorbierenden Polymerpartikeln auf Basis nachwachsender Rohstoffe hergestellt werden ohne auf den Kostenvorteil großer Produktionsanlagen verzichten zu müssen.

Die wasserabsorbierenden Polymerpartikel werden beispielsweise durch Polymerisation einer Monomerlösung oder -suspension, enthaltend
a) Acrylsäure, die zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
hergestellt und sind üblicherweise wasserunlöslich.

Der Anteil an Acrylsäure und/oder deren Salzen an der Gesamtmenge der Monomeren beträgt vorzugsweise mindestens 50 mol-%, besonders bevorzugt mindestens 90 mol-%, ganz besonders bevorzugt mindestens 95 mol-%.

Die eingesetzte Acrylsäure enthält üblicherweise Polymerisationsinhibitoren, vorzugsweise Hydrochinonhalbether, als Lagerstabilisator.

Die Monomerlösung enthält daher vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf die unneutralisierte Acrylsäure. Beispielsweise kann zur Herstellung der Monomerlösung eine Acrylsäure mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen der Acrylsäure kovalente Bindungen ausbilden können. Weiterhin sind auch polyvalente Metallsalze, die mit mindestens zwei Säuregruppen der Acrylsäure koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,2 bis 0,6 Gew.-%, jeweils bezogen auf Acrylsäure. Mit steigendem Vernetzergehalt sinkt die Zentrifugenretentionskapazität (CRC) und die Absorption unter einem Druck von 21,0 g/cm² durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Mit Acrylsäure copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Weitere geeignete Monomere d) sind beispielsweise ethylenisch ungesättigte Carbonsäuren, wie Methacrylsäure und Itaconsäure, und ethylenisch ungesättigte Sulfonsäuren, wie Styrolsulfonsäure und 2-Acrylamido-2-methylpropansulfonsäure (AMPS).

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssiger Acrylsäure, beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur T_{g} auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

In einer bevorzugten Ausführungsform der vorliegenden Erfindung wird eine wässrige Monomerlösung vertropft und die erzeugten Tropfen in einem erwärmten Trägergasstrom polymerisiert. Hierbei können die Verfahrensschritte Polymerisation und Trocknung zusammengefasst werden, wie in WO 2008/040715 A2, in WO 2008/052971 A1 und insbesondere in WO 2011/026876 A1 beschrieben. Bei dieser bevorzugten Ausführungsform wird die Partikelgröße über die Größe der erzeugten Tropfen eingestellt.

Die mittlere Partikelgröße der wasserabsorbierenden Polymerpartikel beträgt vorzugsweise mindestens 200 µm, besonders bevorzugt von 250 bis 600 µm, ganz besonders von 300 bis 500 µm. Die mittlere Partikelgröße kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von größer 150 µm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 µm, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von 150 bis 850 µm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Hydroxid, Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Citrat und Lactat, möglich. Es sind auch Salze mit unterschiedlichen Gegenionen möglich, beispielsweise basische Aluminiumsalze, wie Aluminiummonoacetat oder Aluminiummonolaktat. Aluminiumsulfat, Aluminiummonoacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die wasserabsorbierenden Polymerpartikel nach der thermischen Trocknung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die wasserabsorbierenden Polymerpartikel auf 20 bis 150°C, vorzugsweise 30 bis 120°C, besonders bevorzugt 40 bis 100°C, ganz besonders bevorzugt 50 bis 80°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Trocknung durchgeführt.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Die wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

## Patentansprüche

1. Verfahren zur Herstellung von wasserabsorbierenden Polymerpartikeln, umfassend die Schritte
i) thermische Spaltung von Bio-Naphtha, basierend auf natürlichen Ölen und/oder Fetten, in Gegenwart von Wasserdampf zu einem Gemisch , enthaltend Propan und Propen,
ii) Abtrennung von Propen und zumindest eines Teiles des Propans aus dem in Schritt i) erhaltenen Gemisch,
iii) Gashasenoxidation der in Schritt ii) erhaltenen Propen/Propan-Mischung zu Acrylsäure und
iv) Polymerisation der in Schritt iii) erhalten Acrylsäure zu wasserabsorbierenden Polymerpartikeln.

2. Verfahren gemäß Anspruch 1, **dadurch gekennzeichnet, dass** das Bio-Naphtha auf Palmöl basiert.

3. Verfahren gemäß Anspruch 1 oder 2, **dadurch gekennzeichnet, dass** die Gasphasenoxidation in Schritt iii) zweistufig durchgeführt wird.

4. Verfahren gemäß einem der Ansprüche 1 bis 3, **dadurch gekennzeichnet, dass** in Schritt i) Naphtha und Bio-Naphtha eingesetzt wird.

5. Verfahren gemäß einem der Ansprüche 1 bis 4, **dadurch gekennzeichnet, dass** in Schritt iii) eine Propen/Propan-Mischung auf Basis von Naphtha und eine Propen/Propan-Mischung auf Basis von Bio-Naphtha eingesetzt wird.

6. Verfahren gemäß einem der Ansprüche 1 bis 5, **dadurch gekennzeichnet, dass** in Schritt iv) Acrylsäure auf Basis von Naphtha und Acrylsäure auf Basis von Bio-Naphtha eingesetzt wird.

7. Verfahren gemäß einem der Ansprüche 1 bis 6, **dadurch gekennzeichnet, dass** die in Schritt iv) eingesetzte Acrylsäure von 0,02 bis 2,0 Gew.-% Propionsäure enthält.

8. Verfahren gemäß einem der Ansprüche 1 bis 7, **dadurch gekennzeichnet, dass** in Schritt iv) von 0,2 bis 0,6 Gew.-% eines Vernetzers, bezogen auf Acrylsäure, eingesetzt werden.

9. Verfahren gemäß einem der Ansprüche 1 bis 8, **dadurch gekennzeichnet, dass** die wasserabsorbierenden Polymerpartikel oberflächennachvernetzt werden.

10. Verfahren gemäß einem der Ansprüche 1 bis 9, **dadurch gekennzeichnet, dass** die wasserabsorbierenden Polymerpartikel mit anorganischen inerten Substanzen beschichtet werden.

## Claims

1. A process for producing water-absorbing polymer particles, comprising the steps of
i) thermally cracking bionaphtha based on natural oils and/or fats in the presence of steam to give a mixture comprising propane and propene,
ii) removing propene and at least some of the propane from the mixture obtained in step i),
iii) gas phase oxidation of the propene/propane mixture obtained in step ii) to give acrylic acid and
iv) polymerizing the acrylic acid obtained in step iii) to give water-absorbing polymer particles.

2. The process according to claim 1, wherein the bionaphtha is based on palm oil.

3. The process according to claim 1 or 2, wherein the gas phase oxidation in step iii) is performed in two stages.

4. The process according to any of claims 1 to 3, wherein naphtha and bionaphtha are used in step i).

5. The process according to any of claims 1 to 4, wherein a propene/propane mixture based on naphtha and a propene/propane mixture based on bionaphtha are used in step iii).

6. The process according to any of claims 1 to 5, wherein acrylic acid based on naphtha and acrylic acid based on bionaphtha are used in step iv).

7. The process according to any of claims 1 to 6, wherein the acrylic acid used in step iv) comprises from 0.02 to 2.0% by weight of propionic acid.

8. The process according to any of claims 1 to 7, wherein from 0.2 to 0.6% by weight of a crosslinker, based on acrylic acid, is used in step iv).

9. The process according to any of claims 1 to 8, wherein the water-absorbing polymer particles are surface postcrosslinked.

10. The process according to any of claims 1 to 9, wherein the water-absorbing polymer particles are coated with inorganic inert substances.

## Revendications

1. Procédé pour la production de particules de polymère absorbant l'eau, comprenant les étapes
i) dissociation thermique de bio-naphta, à base d'huiles naturelles et/ou de graisses naturelles, en présence de vapeur d'eau, pour l'obtention d'un mélange contenant du propane et du propène,
ii) séparation du propène et d'au moins une partie du propane à partir du mélange obtenu dans l'étape i),
iii) oxydation en phase gazeuse du mélange de propène/ propane obtenu dans l'étape ii), pour l'obtention d'acide acrylique et
iv) polymérisation de l'acide acrylique obtenu dans l'étape iii), pour l'obtention de particules de polymère absorbant l'eau.

2. Procédé selon la revendication 1, **caractérisé en ce que** le bio-naphta est à base d'huile de palme.

3. Procédé selon la revendication 1 ou 2, **caractérisé en ce que** l'oxydation en phase gazeuse dans l'étape iii) est effectuée en deux stades.

4. Procédé selon l'une quelconque des revendications 1 à 3, **caractérisé en ce que** dans l'étape i) on utilise du naphta et du bio-naphta.

5. Procédé selon l'une quelconque des revendications 1 à 4, **caractérisé en ce que** dans l'étape iii) on utilise un mélange de propène/propane à base de naphta et un mélange de propène/propane à base de bio-naphta.

6. Procédé selon l'une quelconque des revendications 1 à 5, **caractérisé en ce que** dans l'étape iv) on utilise de l'acide acrylique à base de naphta et de l'acide acrylique à base de bio-naphta.

7. Procédé selon l'une quelconque des revendications 1 à 6, **caractérisé en ce que** l'acide acrylique utilisé dans l'étape iv) contient de 0,02 à 2,0 % en poids d'acide propionique.

8. Procédé selon l'une quelconque des revendications 1 à 7, **caractérisé en ce que** dans l'étape iv) on utilise de 0,2 à 0,6 % en poids d'un agent de réticulation, par rapport à l'acide acrylique.

9. Procédé selon l'une quelconque des revendications 1 à 8, **caractérisé en ce qu'**on soumet les particules de polymère absorbant l'eau à une post-réticulation superficielle.

10. Procédé selon l'une quelconque des revendications 1 à 9, **caractérisé en ce qu'**on enrobe avec des substances inorganiques inertes les particules de polymère absorbant l'eau.
